# EUROPÄISCHE PATENTSCHRIFT

(11) **EP 2 731 669 B1**
(45) Veröffentlichungstag und Bekanntmachung des Hinweises auf die Patenterteilung: **22.03.2017**
(21) Anmeldenummer: 12738423.8
(22) Anmeldetag: 10.07.2012
(51) Int. Cl.: A61N 1/05

(54) **IMPLANTIERBARE NERVENELEKTRODE UND VERFAHREN ZUR HERSTELLUNG EINER IMPLANTIERBAREN NERVENELEKTRODE**
IMPLANTABLE NERVE ELECTRODE AND METHOD FOR PRODUCING AN IMPLANTABLE NERVE ELECTRODE
ÉLECTRODE DE NERF IMPLANTABLE ET PROCÉDÉ DE FABRICATION D'UNE ÉLECTRODE DE NERF IMPLANTABLE

(30) Priorität: 11.07.2011 DE 102011078982
(43) Veröffentlichungstag der Anmeldung: 21.05.2014
(73) Patentinhaber: CorTec GmbH, 79110 Freiburg (DE)
(72) Erfinder: SCHÜTTLER, Martin, 79102 Freiburg (DE); KOHLER, Fabian, 79098 Freiburg (DE)
(74) Vertreter: 2s | ip Schramm Schneider Patentanwälte Rechtsanwälte
(86) Internationale Anmeldenummer: PCT/EP2012/063463
(87) Internationale Veröffentlichungsnummer: WO 2013/007718

(56) Entgegenhaltungen:
- WO-A1-2009/003182
- US-A1- 2007 128 420
- US-A1- 2011 034 977
- US-A1- 2011 071 596
- SUANING G J ET AL: "Fabrication of multi-layer, high-density micro-electrode arrays for neural stimulation and bio-signal recording", NEURAL ENGINEERING, 2007. CNE '07. 3RD INTERNATIONAL IEEE/EMBS CONFERENCE ON, IEEE, PISCATAWAY, NJ, USA, 1. Mai 2007 (2007-05-01), Seiten 5-8, XP031174665, DOI: 10.1109/CNE.2007.369597 ISBN: 978-1-4244-0791-0

## Beschreibung

Die Erfindung betrifft eine implantierbare Nervenelektrode gemäß Anspruch 1 sowie ein Verfahren zur Herstellung einer solchen implantierbaren Nervenelektrode.

Funktionelle elektrische Stimulation stellt für viele Patienten mit implantierten Vorrichtungen, wie beispielsweise Herzschrittmachern, Defibrillatoren, Blasenstimulatoren, Implantaten zur Schmerzbewältigung, Tremor, Epilepsie und zur Wiederherstellung des Gehörs, verloren gegangene Körperfunktionen wieder hier. Hierzu werden implantierbare Nervenelektroden eingesetzt.

Im Stand der Technik ist es bekannt, implantierbare Nervenelektroden mittels Laserbehandlung aus medizinischem Silikon und einer Metallfolie herzustellen. Die Grundlage der bekannten Verfahren ist das Heraustrennen von Leiterbahnen und Kontaktflächen aus einer typischerweise 5 bis 25 µm dünnen Metallfolie mittels eines Lasers. Die Leiterbahnen, Elektroden- und Kontaktflächen werden im medizinischen Silikon eingebettet, wodurch die einzelnen Signalwege elektrisch voneinander isoliert werden können. Die Kontaktflächen werden anschließend mit dem Laser freigelegt.

Diese bekannte Technologie weist jedoch zwei Probleme auf: Die Leiterbahnen sind aufgrund ihrer Feinheit äußerst fragil. Das elastische Silikon kann sie nur sehr bedingt vor mechanischen Einwirkungen, wie sie beispielsweise bei der Handhabung während der Implantation auftreten, schützen. Dementsprechend sind laserprozessierte Nervenelektroden einerseits relativ anfällig für Leiterbahnbrüche. Andererseits weist Silikon eine geringe Durchschlagfestigkeit auf. Wenn mit Nervenelektroden elektrisch stimuliert wird, kann es zu Spannungen von einigen 10 V zwischen zwei benachbarten Leiterbahnen kommen. Nach Angaben der Hersteller kann die Durchschlagfestigkeit von Silikon durch Lagerung in Wasser auf 2 kV/mm sinken. Eine Spannung von 20 V zwischen zwei benachbarten Leiterbahnen mit einem Abstand von 10 µm kann folglich zu einem elektrischen Durchschlag führen. Dieser Sachverhalt limitiert die Integrationsdichte der Elektrode.

Um das Problem der mangelhaften Stabilität zu lösen, ist es im Stand der Technik bekannt, beispielsweise die Leiterbahnen in Form von Schlangenlinien anzuordnen. Hierdurch wird eine gewisse Dehnbarkeit der Leiterbahnen erreicht. Nachteilig bei der schlangenförmigen Anordnung der Leiterbahnen ist jedoch der erhöhte Platzbedarf, was sich wiederum negativ auf die maximale Integrationsdichte auswirkt.

Ein weiterer Lösungsansatz sieht die Einbettung der Leiterbahnen in dickerem und härterem Silikon vor. Jedoch zeigt auch diese Variante nur einen geringen Erfolg, da auch festeres Silikon sehr viel elastischer als das darin eingebettete Metall ist. Die auftretenden mechanischen Kräfte wirken weiterhin im Wesentlichen auf die Leiterbahnen und führen zu deren Beschädigung.

Auch wurde versucht, die mechanische Stabilität durch Einzufügen einer mechanisch hochfesten Polymerfolie und einer weiteren Silikonschicht, welche in den Schichtverbund aus Silikon-Metall-Silikon eingefügt wurden, zu erhöhen. Hierdurch kann zwar eine Verbesserung der mechanischen Stabilität der Nervenelektrode erzielt werden, jedoch der neue Schichtverbund Silikon-Metall-Silikon-Polymerfolie-Silikon bring den Dachteil mit sich, dass die Lage der festen, nicht stauch- oder dehnbaren Polymerfolie die mechanisch neutrale Faser im Schichtverbund definiert. Bei starken Biegebewegungen kommt es deshalb zu Stauchungen oder Zugbelastungen der Metallleiterbahnen.

Was das zweite Problem der geringen elektrischen Durchschlagfestigkeit des Silikons, in welches die Leiterbahnen eingebettet sind, angeht, wurde bisher noch kein Lösungsansatz gefunden.

US 2011/0034977A1 bezieht sich auf ein implantierbares Elektrodenarray, das mehrere beabstandete Elektroden aufweist, zu denen Strom hin- und angeführt werden kann. Das Feld umfasst einen Träger, der die Elektroden trägt. Eines oder mehrere Steuermodule, die Strom zu den Elektroden hin- oder von ihnen zuführen, sind in dem Träger angeordnet. Eine Materialschicht, die flexibler ist als der Träger, ist zwischen dem Träger und dem Steuermodul einerseits und den Elektroden andererseits angeordnet. Leiter, über welche Befehle und Energie den Steuermodulen zugeführt werden, und Leiter, welche sich zwischen den Steuermodulen und den Elektroden erstrecken, sind eingebettet in und verlaufen durch die Schicht des flexiblen Materials hindurch.

SUANING G J ET AL: "Fabrication of multi-layer, high-density micro-electrode arrays for neural Stimulation and bio-signal recording", NEURAL ENGINEERING, 2007, CNE '07. 3RD INTERNATIONAL IEEE/EMBS CONFERENCE ON, IEEE, PISCATAWAY, NJ, USA, 1. Mai 2007 (2007-05-01), Seiten 5-8, XP031174665, DOI: 10.1109/CNE.2007.369597 ISBN: 978-1-4244-0791-0 betrifft die Herstellung von mehrschichtigen, hochintegrierten Mikoelektrodenarrays für neuronale Stimulierung und Biosignalaufzeichnung. Es werden Mikro-gefertigte Bauteile unter Verwendung von Laserabtragungsverfahren des Edelmetalls Platin und des Elastomers Silikon und von PDMS beschrieben.

Daher ist es eine Aufgabe der vorliegenden Erfindung, eine implantierbare Nervenelektrode und ein Verfahren zur Herstellung einer Nervenelektrode bereitzustellen, bei welchen ein effektiver Schutz für die Leiterbahnen und gleichzeitig eine hohe Integrationsdichte erzielt werden können.

Diese Aufgabe wird durch eine implantierbare Nervenelektrode mit den Merkmalen gemäß Anspruch 1 und durch ein Verfahren zum Herstellen einer Nervenelektrode mit den Merkmalen gemäß Anspruch 8 gelöst. Vorteilhafte Weiterbildungen der Erfindung sind in den jeweiligen abhängigen Ansprüchen definiert.

Gemäß der Erfindung wird eine implantierbare Nervenelektrode bereitgestellt, welche ein elektrisch isolierendes Substrat mit darin verlaufenden Leiterbahnen, Elektrodenkontakten und Anschlusskontakten aufweist, wobei die Leiterbahnen die Elektrodenkontakte mit den Anschlusskontakten verbinden, und wobei die Elektrodenkontakte mit Nerven eines Nervensystems verbindbar sind, wobei jede der Leiterbahnen eine zumindest teilweise Ummantelung aus einem mechanisch festen und elektrisch gut isolierenden Polymer aufweist.

Dadurch, dass jede Leiterbahn innerhalb des Verbundes Silikon-Metall-Silikon individuell von einem mechanisch festen und elektrisch gut isolierenden Polymer zumindest teilweise ummantelt ist, können einerseits die Nachteile bezüglich der mangelhaften mechanischen Stabilität der Leiterbahnen behoben werden. Andererseits kann eine hohe Integrationsdichte erzielt werden, da die Ummantelung eine gute elektrische Isolierung sicherstellt. Durch die erfindungsgemäße Konfiguration liegt die mechanisch neutrale Faser in der Ebene des Metalls, wodurch es auch bei starker Biegebeanspruchung nicht zu Stauchung oder Dehnung der Leiterbahnen kommt. Für einen Patienten, der eine Nervenelektrode implantiert bekommt, kann durch die höhere mechanische Stabilität eine höhere Zuverlässigkeit des Implantats sichergestellt werden. Auch ermöglicht die elektrische Isolierung zwischen den einzelnen Leiterbahnen der Nervenelektrode eine weitere Miniaturisierung der in den Körper eines Patienten einzubringenden Struktur als auch die Realisierung von komplexeren Systemen mit höherer Integrationsdichte.

Gemäß einer bevorzugten Ausführungsform umfasst das mechanisch feste und elektrisch gut isolierende Polymer, aus welchem die Ummantelung hergestellt ist, Parylene, insbesondere Parylen C, Polyethylen oder Polypropylen. Diese Materialien haben eine ca. 100 mal höhere Durchschlagfestigkeit als das bisher verwendete Silikon. Insbesondere Parylen C hat eine Durchschlagfestigkeit von 220 kV/mm, was eine erheblich verbesserte Integrationsdichte der Leiterbahnen erlaubt.

Gemäß einer weiteren bevorzugten Ausführungsform sind die Leiterbahnen, die Elektrodenkontakte und die Anschlusskontakte aus einer laserstrukturierten Metallfolie hergestellt. Die Herstellung der Leiterbahnen, Elektroden- und Anschlusskontakte mittels Laser ermöglicht eine besonders gute Reproduzierbarkeit und ein hohes Maß an Automatisierung.

Erfindungsgemäß vorgesehen ist, dass jede einzelne der Leiterbahnen eine individuelle Ummantelung aufweist. Diese Konfiguration bietet einen besonders effektiven Schutz der fragilen Leiterbahnen.

Vorzugsweise ist jede der Leiterbahnen vollständig von der Ummantelung umschlossen, was den Schutz der Leiterbahnen noch weiter verbessert.

Gemäß noch einer weiteren bevorzugten Ausführungsform ist das mechanisch feste und elektrisch gut isolierende Polymer zusätzlich zwischen den Leiterbahnen vorhanden. Hierdurch kann gezielt lokal eine mechanische Verstärkung der Nervenelektrode realisiert werden.

Vorzugsweise sind die mit dem mechanisch festen und elektrisch gut isolierenden Polymer ummantelten Leiterbahnen in Silikon, insbesondere in medizinischen Silikon, insbesondere Polydimethylsiloxan, eingebettet, welches das elektrisch isolierende Substrat der implantierbaren Nervenelektrode bildet.

Gemäß einer weiteren bevorzugten Ausführungsform sind die Ummantelungen aus dem mechanisch festen und elektrisch gut isolierenden Polymer miteinander verbunden, um so das elektrisch isolierende Substrat der implantierbaren Nervenelektrode zu bilden. Hierbei ist von Vorteil, dass auf weitere Schichten, die Silikonschichten, verzichtet werden kann. Das Herstellungsverfahren einer derartigen Nervenelektrode wird dadurch entsprechend vereinfacht und somit kostengünstiger,

Darüber hinaus ist es bevorzugt, wenn die Leiterbahnen, die Elektrodenkontakte und die Anschlusskontakte aus rostfreiem Stahl oder aus Platin hergestellt sind.

Gemäß der Erfindung wird ein Verfahren zum Herstellen einer implantierbaren Nervenelektrode bereitgestellt, wobei das Verfahren die folgenden Schritte umfasst: Vorsehen eines mechanischen Trägers: Aufbringen einer Antihaft-Beschichtung auf eine Oberseite des mechanischen Trägers (wenn erforderlich), Aufbringen einer ersten Silikonschicht auf die Oberseite des mechanischen Trägers, Auflaminieren einer einseitig mit einer ersten Schicht aus einem mechanisch festen und elektrisch gut isolierenden Polymer beschichteten Metallfolie, wobei die beschichtete Seite der Metallfolie der ersten Silikonschicht gegenüberliegt, Strukturieren der Metallfolie mittels eines Lasers, um Leiterbahnen, Elektrodenkontakte und Anschlusskontakte freizulegen, Aufbringen einer Deckschicht aus dem mechanisch festen und elektrisch gut isolierenden Polymer auf die strukturierte Metallfolie, wobei sich die Deckschicht mit der ersten Schicht aus einem mechanisch festen und elektrisch gut isolierenden Polymer verbindet. Durch das erfindungsgemäße Verfahren werden die oben beschriebenen Vorteile erzielt. Insbesondere kann hierdurch eine Nervenelektrode mit verbesserter mechanischer Stabilität und höherer Integrationsdichte der Leiterbahnen hergestellt werden. Der erfindungsgemäße Fertigungsprozess ermöglicht darüber hinaus eine lokal definierbare und gegebenenfalls anisotrope Steifigkeit der Nervenelektrode, die für die jeweilige Anwendung angepasst werden kann.

Gemäß einer bevorzugten Ausführungsform umfasst das Verfahren weiterhin den Schritt des Strukturierens der Deckschicht mittels Laser.

Gemäß noch einer bevorzugten Ausführungsform umfasst das Verfahren weiterhin den Schritt des Aufbringens einer zweiten Silikonschicht, insbesondere des Aufschleuderns von flüssigem Silikon, auf die strukturierte Deckschicht.

Gemäß noch einer weiteren bevorzugten Ausführungsform umfasst das Verfahren weiterhin den Schritt des Aushärtens der zweiten Silikonschicht.

Vorzugsweise umfasst das Verfahren weiterhin den Schritt des Freilegens der Elektrodenkontakte und der Anschlusskontakte mittels Laser.

Gemäß noch einer weiteren bevorzugten Ausführungsform umfasst das Verfahren weiterhin den Schritt des Definierens der äußeren Konturen der implantierbaren Nervenelektrode mittels Laser.

Vorzugsweise umfasst das Verfahren weiterhin den Schritt des Lösens der mittels Laser definierten implantierbaren Nervenelektrode von dem Träger.

Gemäß noch einer weiteren bevorzugten Ausführungsform ist die Antihaft-Beschichtung eine PVC-Folie, insbesondere Tesafilm.

Im Nachfolgenden werden Ausführungsbeispiele der Erfindung unter Bezugnahme auf die beigefügte Zeichnung näher beschrieben. Es zeigen:
- Fig. 1: eine perspektivische Ansicht einer implantierbaren Nervenelektrode gemäß dem Stand der Technik;
- Fig. 2a bis 2p: jeweilige Schnittansichten der Abfolge von Verfahrensschritten eines Verfahrens zur Herstellung einer implantierbaren Nervenelektrode gemäß einer Ausführungsform der Erfindung; und
- Fig. 3a bis 3n: jeweilige Schnittansichten der Abfolge von Verfahrensschritten eines Verfahrens zur Herstellung einer implantierbaren Nervenelektrode gemäß einer weiteren Ausführungsform der Erfindung.

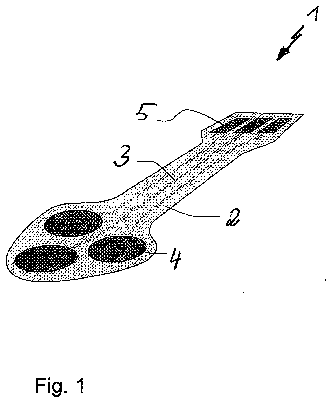

Figur 1 ist eine perspektivische Ansicht einer implantierbaren Nervenelektrode 1 gemäß dem Stand der Technik. Die Nervenelektrode 1 umfasst ein elektrisch isolierendes Substrat 2, welches hier medizinisches Silikon ist, in dem Leiterbahnen 3 eingebettet sind. Die Leiterbahnen 3 verbinden Elektrodenkontakte 4 mit Anschlusskontakten 5.

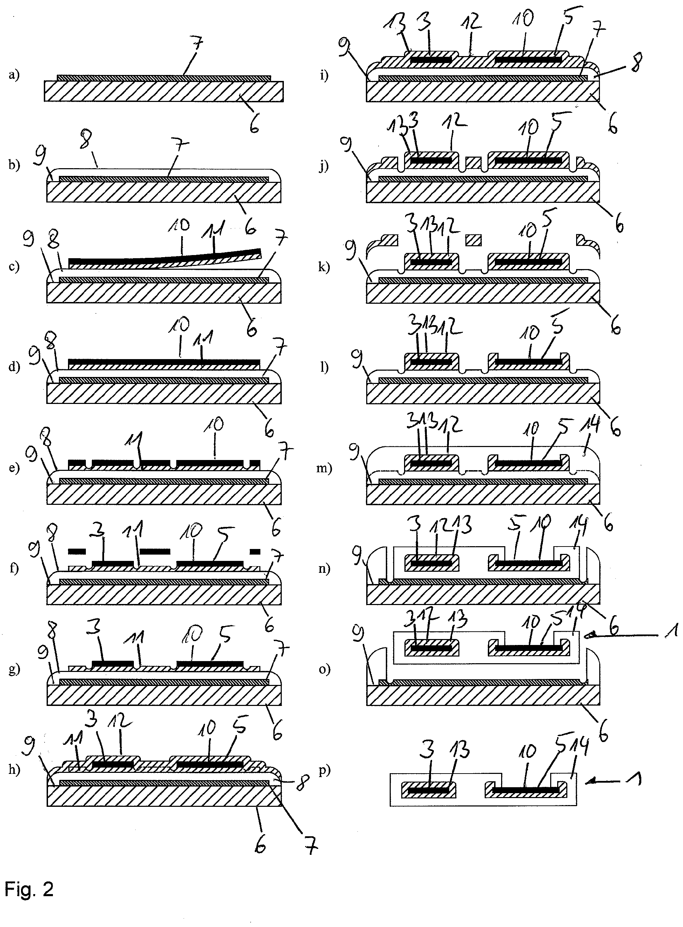

Fig. 2a bis 2p sind jeweilige Schnittansichten der Abfolge von Verfahrensschritten eines Verfahrens zur Herstellung einer implantierbaren Nervenelektrode gemäß einer Ausführungsform. In Fig. 2a ist der erste Verfahrensschritt dargestellt, in welchem auf einen mechanischen Träger 6, der beispielsweise aus Glas oder Keramik besteht, eine Antihaft-Beschichtung 7, wie z.B. selbstklebende PVC-Folie, wie beispielsweise Tesafilm, aufgebracht wird. Im zweiten, in Fig. 2b dargestellten Schritt, wird eine nur wenige 10 Mikrometer dünne erste Silikonschicht 8 aus flüssigem Silikon auf die Oberseite 9 des bereits mit der Antihaft-Beschichtung 7 versehenen Trägers 6 aufgeschleudert und anschließend ausgehärtet. Danach wird, wie in Fig. 2c und 2d dargestellt ist, eine Metallfolie 10, welche in der Ausführungsform eine Dicke von 12,5 µm aufweist und aus Platin hergestellt ist, und welche einseitig mit einer mit einer nur wenige Mikrometer dicken ersten Schicht 11 aus einem mechanisch festen und elektrisch gut isolierenden Polymer, in der Ausführungsform Parylen C, versehen ist, auf die erste Silikonschicht 8 auflaminiert. Im darauffolgenden Schritt, der in Fig. 2e dargestellt ist, wird die Metallfolie 10 mittels eines Lasers strukturiert, so dass das Metall, welches später nicht als Leiterbahn 3, Elektroden- oder Anschlusskontakt (4, 5; siehe Fig. 1) dient, entfernt werden kann, wie in Fig. 2f und 2g erkennbar ist. Dann wird, wie in Fig. 2h dargestellt ist, eine Deckschicht 12, welche nur wenige Mikrometer dick ist und ebenfalls aus Parylen C besteht, aufgebracht, die sich mit der ersten Schicht 11 verbindet (Fig. 2i), und für jede Leiterbahn 3 eine individuelle Ummantelung 13 gebildet wird. In nächsten Schritt, der in Fig. 2j dargestellt ist, werden mit einem Laser die äußeren Konturen der späteren Parylenschicht, nämlich der Deckschicht 12, strukturiert und das überschüssige Parylen wird entfernt (Fig. 2k). In einem weiteren Laserschritt, der in Fig. 2l gezeigt ist, wird die Deckschicht 12 an den Stellen der späteren Elektroden, hier dargestellt ist die Anschlusselektrode 5, entfernt. Durch Aufschleudern einer zweiten Silikonschicht 14, die nur wenige Mikrometer dick ist, und anschließendem Aushärten werden die Parylen-ummantelten Leiterbahnen 3 komplett in Silikon eingegossen. Mit einem Laser werden dann die Öffnungen für Elektroden- und Anschlusskontakte, hier dargestellt der Anschlusskontakt 4, in das Silikon geschnitten (Fig. 2n) und die äußeren Konturen der Nervenelektrode 1 definiert. Aufgrund der schlechten Haftung zwischen Silikon und der Antihaft-Beschichtung 7 kann nun die Nervenelektrode 1 vom mechanischen Träger 6 gelöst werden, wie in Fig. 2o erkennbar ist. In Fig. 2p ist schließlich die am Ende des Fertigungsprozesses hergestellte Nervenelektrode 1 gezeigt.

Es ist jedoch gemäß einer weiteren Ausführungsform ebenfalls möglich, eine Beschichtung mit der zweiten Silikonschicht 14 wegzulassen. Dabei werden dann die in Fig. 2i und 2j dargestellten Verfahrensschritte derartig ausgeführt, dass alle Leiterbahnen 3 über ihre Ummantelungen 13 aus Parylen C mechanisch miteinander verbunden sind und nur die äußere Kontur der Nervenelektrode durch Laser-Schnitte in das Polymer definiert wird. Entsprechend des in Fig. 2l dargestellten Verfahrensschrittes werden dann die Elektroden- und Anschlusskontakte mittels Laser freigelegt und die fertige Nervenelektrode 1 kann anschließend von der ersten Silikonschicht 8 abgezogen werden. Die so gefertigte Nervenelektrode 1 weist gemäß diesem Herstellungsprozess Leiterbahnen 3 und Elektroden- und Anschlusskontakte 4, 5 auf, welche in einem Substrat aus Parylen C eingebettet sind.

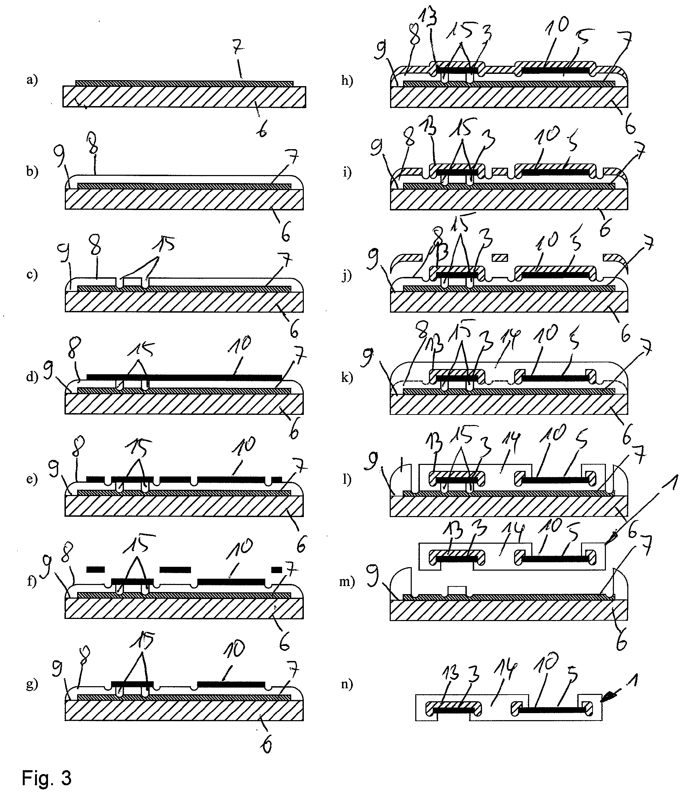

Fig. 3a bis 3n sind jeweilige Schnittansichten der Abfolge von Verfahrensschritten eines Verfahrens zur Herstellung einer implantierbaren Nervenelektrode 1 gemäß einer weiteren Ausführungsform, gemäß die Leiterbahnen 3 nur teilweise mit einem mechanisch festen und elektrisch gut isolierenden Polymer, hier ebenfalls Parylen C, ummantelt sind. Hierdurch wird eine Vereinfachung des Herstellungsverfahrens erzielt und eine Möglichkeit geschaffen, eine elektrische Öffnung nach "unten" zu realisieren, wie im Folgenden ersichtlich wird. Die in Fig. 3a und 3b dargestellten Verfahrensschritte entsprechen denen in Fig. 2a und 2b und werden daher nicht wiederholt beschrieben. In dem in Fig. 3c dargestellten Schritt werden die Umrandungen von späteren Elektrodenöffnungen gelasert, wie durch das Bezugszeichen 15 angedeutet ist. Dann wird, wie in Fig. 3d gezeigt ist, eine Metallfolie 10 auf die erste Silikonschicht 8 auflaminiert, welche anschließend mittels eines Lasers so geschnitten wird, dass die nicht als Leiterbahnen 3, Elektroden- oder Anschlusskontakte 4, 5 (siehe Fig. 1) benötigten Bereiche entfernt werden (Fig. 3e, 3f, 3g). Anschließend wird Parylen C flächig aufgebracht, um eine teilweise Ummantelung 13 für die Leiterbahn 5 zu bilden (Fig. 3h). In Fig. 3i ist der nächste Schritt dargestellt, in welchem die Schicht aus Parylen C mittels Laser strukturiert wird, so dass unerwünscht beschichtete Bereiche anschließend von Parylen C befreit werden können (Fig. 3j). Anschließend wird eine Silikonschicht 14 aufgebracht (Fig. 3k), welche ausgehärtet wird und dann derart bearbeitet wird (Fig. 3l), dass nach "oben" zeigende Elektroden- und Anschlusskontakte, hier der Anschlusskontakt 5, freigelegt werden und die äußere Umrandung der Nervenelektrode 1 definiert wird. Zum Abschluss des Herstellungsverfahrens wird der mechanische Träger 6 entfernt (Fig. 3m), so dass die fertige Nervenelektrode 1 erhalten wird (Fig. 3n).

### Bezugszeichenliste

- 1: Nervenelektrode
- 2: elektrisch isolierendes Substrat
- 3: Leiterbahn
- 4: Elektrodenkontakt
- 5: Anschlusskontakt
- 6: Träger
- 7: Antihaft-Beschichtung
- 8: erste Silikonschicht
- 9: Oberseite des Trägers
- 10: Metallfolie
- 11: erste Schicht
- 12: Deckschicht
- 13: Ummantelung
- 14: zweite Silikonschicht
- 15: Umrandung

## Patentansprüche

1. Implantierbare Nervenelektrode (1), welche ein elektrisch isolierendes Substrat (2) mit darin verlaufenden Leiterbahnen (3), Elektrodenkontakten (4) und Anschlusskontakten (5) aufweist, wobei die Leiterbahnen (3) die Elektrodenkontakte (4) mit den Anschlusskontakten (5) verbinden, und wobei die Elektrodenkontakte (4) mit Nerven eines Nervensystems verbindbar sind, wobei jede der Leiterbahnen (3) eine zumindest teilweise Ummantelung (13) aus einem mechanisch festen und elektrisch gut isolierenden Polymer aufweist,
wobei die Leiterbahnen (3), die Elektrodenkontakte (4) und die Anschlusskontakte (5) aus einer laserstrukturierten Metallfolie (10) hergestellt sind,
wobei jede einzelne der Leiterbahnen (3) eine individuelle Ummantelung (13) aufweist und
wobei die mit dem Polymer ummantelten Leiterbahnen (3) in Silikon eingebettet sind, welches das elektrisch isolierende Substrat (2) der implantierbaren Nervenelektrode (1) bildet.

2. Implantierbare Nervenelektrode (1) gemäß Anspruch 1, wobei das Polymer Parylen, insbesondere Parylen C, Polyethylen oder Polypropylen umfasst.

3. Implantierbare Nervenelektrode (1) gemäß einem der Ansprüche 1 bis 2, wobei jede der Leiterbahnen (3) vollständig von der Ummantelung (13) umschlossen ist.

4. Implantierbare Nervenelektrode (1) gemäß einem der Ansprüche 1 bis 3, wobei das Polymer zusätzlich zwischen den Leiterbahnen (3) vorhanden ist.

5. Implantierbare Nervenelektrode (1) gemäß einem der Ansprüche 1 bis 4, wobei das Silikon, in welchem die mit dem Polymer ummantelten Leiterbahnen (3) eingebettet sind, ein medizinisches Silikon, insbesondere ein Polydimethylsiloxan, ist.

6. Implantierbare Nervenelektrode (1) gemäß einem der Ansprüche 1 bis 4, wobei die Ummantelungen (13) aus dem Polymer miteinander verbunden sind, um das elektrisch isolierende Substrat (2) der implantierbaren Nervenelektrode (1) zu bilden.

7. Implantierbare Nervenelektrode (1) gemäß einem der Ansprüche 1 bis 6, wobei die Leiterbahnen (3), die Elektrodenkontakte (4) und die Anschlusskontakte (5) aus Metall, insbesondere aus rostfreiem Stahl oder aus Platin, hergestellt sind.

8. Verfahren zum Herstellen einer implantierbaren Nervenelektrode (1) gemäß irgendeinem der Ansprüche 1 bis 7, wobei das Verfahren die folgenden Schritte umfasst:
- Vorsehen eines mechanischen Trägers (6),
- Aufbringen einer Antihaft-Beschichtung (7) auf eine Oberseite (9) des mechanischen Trägers (6),
- Aufbringen einer ersten Silikonschicht (8) auf die Oberseite (9) des mechanischen Trägers (6),
- Auflaminieren einer einseitig mit einer ersten Schicht (11) aus einem mechanisch festen und elektrisch gut isolierenden Polymer beschichteten Metallfolie (10), wobei die beschichtete Seite der Metallfolie (10) der ersten Silikonschicht (8) gegenüberliegt,
- Strukturieren der Metallfolie (10) mittels eines Lasers, um Leiterbahnen (3), Elektrodenkontakte (4) und Anschlusskontakte (5) freizulegen,
- Aufbringen einer Deckschicht (12) aus einem Polymer auf die strukturierte Metallfolie (10), wobei sich die Deckschicht (12) mit der ersten Schicht (11) aus Polymer verbindet,
weiterhin den Schritt umfassend des Aufbringens einer zweiten Silikonschicht (14) auf die strukturierte Deckschicht (12).

9. Verfahren gemäß Anspruch 8, welches weiterhin den Schritt umfasst des Strukturierens der Deckschicht (12) mittels Laser.

10. Verfahren gemäß Anspruch 8 oder 9, wobei der Schritt des Aufbringens einer zweiten Silikonschicht (14) ein Aufschleudern von flüssigem Silikon ist.

11. Verfahren gemäß einem der Ansprüche 8 bis 10, welches weiterhin den Schritt des Aushärtens der zweiten Silikonschicht umfasst.

12. Verfahren gemäß einem der Ansprüche 8 bis 11, welches weiterhin den Schritt umfasst des Freilegens der Elektrodenkontakte (4) und der Anschlusskontakte (5) mittels Laser.

13. Verfahren gemäß einem der Ansprüche 8 bis 12, welches weiterhin den Schritt umfasst des Definierens der äußeren Konturen der implantierbaren Nervenelektrode (1) mittels Laser.

14. Verfahren gemäß einem der Ansprüche 8 bis 13, welches weiterhin den Schritt umfasst des Lösens der mittels Laser definierten implantierbaren Nervenelektrode (1) von dem Träger (6).

15. Verfahren gemäß einem der Ansprüche 8 bis 14, bei welchen als Antihaft-Beschichtung (7) eine PVC-Folie verwendet wird.

## Claims

1. An implantable nerve electrode (1) which has an electrically insulating substrate (2) with strip conductors (3), electrode contacts (4), and connection contacts (5) extending therein, said strip conductors (5) interconnecting said electrode contacts (4) and said connection contacts (5), and said electrode contacts (4) being connectable with the nerves of a nervous system, each of the strip conductors (3) having an at least partially formed envelope (13) made of a mechanically resistant and electrically well-insulating polymer,
said strip conductors (3), electrode contacts (4), and connection contacts (5) being made of a laser-structured metal foil (10),
each of said strip conductors (3) having its own, individual envelope (13), and
the polymer-enveloped strip conductors (3) being embedded in silicone, which forms the electrically insulating substrate (2) of the implantable nerve electrode (1).

2. The implantable nerve electrode (1) as claimed in claim 1, wherein said polymer comprises parylenes, in particular parylene C, polyethylene, or polypropylene.

3. The implantable nerve electrode (1) as claimed in any one of claims 1 to 2, wherein each of the strip conductors (3) is entirely enclosed by the envelope (13).

4. The implantable nerve electrode (1) as claimed in any one of claims 1 to 3, wherein the polymer is additionally present between the strip conductors (3).

5. The implantable nerve electrode (1) as claimed in any one of claims 1 to 4, wherein the silicone in which the polymer-enveloped strip conductors (3) are embedded is a medical-grade silicone, in particular a polydimethylsiloxane.

6. The implantable nerve electrode (1) as claimed in any one of claims 1 to 4, wherein the polymer envelopes (13) are interconnected, thus forming the electrically insulating substrate (2) of the implantable nerve electrode (1).

7. The implantable nerve electrode (1) as claimed in any one of claims 1 to 6, wherein said strip conductors (3), electrode contacts (4), and connection contacts (5) are made of metal, in particular of stainless steel, or of platinum.

8. A method of fabricating an implantable nerve electrode (1) as claimed in any one of claims 1 to 7, wherein the method comprises the steps of:
- providing a mechanical support (6),
- applying a non-stick coating (7) onto an upper surface (9) of the mechanical support (6),
- applying a first silicone layer (8) onto the upper surface (9) of the mechanical support (6),
- applying, by a laminating operation, a metal foil (10) coated one one side with a first layer (11) made of a mechanically resistant and electrically well-insulating polymer, the coated side of the metal foil (10) facing towards the first silicone layer (8),
- structuring the metal foil (10) with a laser device in order to expose the strip conductors (3), electrode contacts (4), and connection contacts (5),
- applying a covering layer (12) made of a polymer onto the structured metal foil (10), said covering layer (12) coalescing with the first polymer layer (11), further comprising the step of applying a second silicone layer (14) onto the structured covering layer (12).

9. The method as claimed in claim 8, further comprising the step of structuring the covering layer (12) using a laser device.

10. The method as claimed in claim 8 or 9, the step of applying a second silicone layer (14) being a spin-coating process of liquid silicone.

11. The method as claimed in any one of claims 8 to 10, further comprising the step of curing the second silicone layer.

12. The method as claimed in any one of claims 8 to 11, further comprising the step of exposing the electrode contacts (4) and the connection contacts (5) using a laser device.

13. The method as claimed in any one of claims 8 to 12, further comprising the step of defining the outer contours of the implantable nerve electrode (1) using a laser device.

14. The method as claimed in any one of claims 8 to 13, further comprising the step of detaching the laser-defined, implantable nerve electrode (1) from the support (6).

15. The method as claimed in any one of claims 8 to 14, wherein a PVC foil is used as a non-stick coating (7).

## Revendications

1. Neuroélectrode implantable (1) qui présente un substrat électro-isolant (2) dans lequel s'étendent des pistes conductrices (3), des contacts d'électrode (4) et des contacts de connexion (5), les pistes conductrices (3) reliant les contacts d'électrode (4) aux contacts de connexion (5) et les contacts d'électrode (4) pouvant être reliés à des nerfs d'un système nerveux, chacune des pistes conductrices (3) présentant une enveloppe (13) au moins partielle, réalisée en un polymère résistant sur le plan mécanique et bien isolant sur le plan électrique, dans laquelle
les pistes conductrices (3), les contacts d'électrode (4) et les contacts de connexion (5) sont fabriqués à partir d'une feuille métallique (10) structurée par laser,
chacune des pistes conductrices (3) présente une enveloppe individuelle (13) et
les pistes conductrices (3) enveloppées dudit polymère sont noyées dans de la silicone qui forme le substrat électro-isolant (2) de la neuroélectrode implantable (1).

2. Neuroélectrode implantable (1) selon la revendication 1, dans laquelle le polymère comprend des parylènes, en particulier du parylène C, du polyéthylène ou du polypropylène.

3. Neuroélectrode implantable (1) selon l'une quelconque des revendications 1 à 2, dans laquelle chacune des pistes conductrices (3) est entièrement entourée par l'enveloppe (13).

4. Neuroélectrode implantable (1) selon l'une quelconque des revendications 1 à 3, dans laquelle le polymère se trouve en outre entre les pistes conductrices (3).

5. Neuroélectrode implantable (1) selon l'une quelconque des revendications 1 à 4, dans laquelle la silicone dans laquelle sont noyées les pistes conductrices (3) enveloppées du polymère est une silicone médicale, en particulier un polydiméthylsiloxane.

6. Neuroélectrode implantable (1) selon l'une quelconque des revendications 1 à 4, dans laquelle les enveloppes (13) constituées par le polymère sont reliées entre elles afin de former le substrat électro-isolant (2) de la neuroélectrode implantable (1).

7. Neuroélectrode implantable (1) selon l'une quelconque des revendications 1 à 6, dans laquelle les pistes conductrices (3), les contacts d'électrode (4) et les contacts de connexion (5) sont fabriqués en métal, en particulier en acier inoxydable ou en platine.

8. Procédé destiné à réaliser une neuroélectrode implantable (1) selon l'une quelconque des revendications 1 à 7, ledit procédé comprenant les étapes suivantes :
- la prévision d'un support mécanique (6),
- l'application d'une couche antiadhésive (7) sur une face supérieure (9) du support mécanique (6),
- l'application d'une première couche de silicone (8) sur la face supérieure (9) du support mécanique (6),
- l'application par lamination d'une feuille métallique (10) revêtue, sur une face, d'une première couche (11) constituée par un polymère résistant sur le plan mécanique et bien isolant sur le plan électrique, la face revêtue de la feuille métallique (10) étant située en face de la première couche de silicone (8),
- la structuration de la feuille métallique (10) au moyen d'un laser afin de mettre à nu des pistes conductrices (3), des contacts d'électrode (4) et des contacts de connexion (5),
- l'application d'une couche de revêtement (12) constituée par un polymère sur la feuille métallique structurée (10), la couche de revêtement (12) s'unissant à la première couche (11) en polymère, ledit procédé comprenant en outre l'étape dans laquelle une deuxième couche de silicone (14) est appliquée sur la couche de revêtement structurée (12).

9. Procédé selon la revendication 8, lequel comprend en outre l'étape dans laquelle la couche de recouvrement (12) est structurée par laser.

10. Procédé selon la revendication 8 ou 9, lors duquel l'étape dans laquelle est appliquée une deuxième couche de silicone (14) consiste en une application par centrifugation de silicone liquide.

11. Procédé selon l'une quelconque des revendications 8 à 10, lequel comprend en outre l'étape dans laquelle s'effectue le durcissement de la deuxième couche de silicone.

12. Procédé selon l'une quelconque des revendications 8 à 11, lequel comprend en outre l'étape dans laquelle les contacts d'électrode (4) et les contacts de connexion (5) sont mis à nu par laser.

13. Procédé selon l'une quelconque des revendications 8 à 12, lequel comprend en outre l'étape qui consiste à définir par laser les contours extérieurs de la neuroélectrode implantable (1).

14. Procédé selon l'une quelconque des revendications 8 à 13, lequel comprend en outre l'étape qui consiste à détacher du support (6) la neuroélectrode implantable (1) définie par laser.

15. Procédé selon l'une quelconque des revendications 8 à 14, lors duquel une feuille PVC est utilisée comme revêtement antiadhésif (7).
